(19) Europäisches Patentamt
European Patent Office
Office européen des brevets

(11) **EP 3 480 615 A1**

(12) **EUROPEAN PATENT APPLICATION**

(43) Date of publication:
**08.05.2019 Bulletin 2019/19**

(51) Int Cl.:
***G01R 33/48*** (2006.01)

(21) Application number: **18198496.4**

(22) Date of filing: **03.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.11.2017 US 201715802767**

(71) Applicant: **General Electric Company Schenectady, NY 12345 (US)**

(72) Inventor: **WIESINGER, Florian 85748 Garching (DE)**

(74) Representative: **Fennell, Gareth Charles et al Kilburn & Strode LLP Lacon London 84 Theobalds Road London WC1X 8NL (GB)**

(54) **SYSTEM AND METHOD FOR MAGNETIC RESONANCE IMAGING AN OBJECT WITH A PLURALITY OF READOUT GRADIENT AMPLITUDES**

(57) A system 10 for magnetic resonance imaging an object 84 with a plurality of readout gradient amplitudes 88, 90, 92 is provided. The system 10 includes a magnet assembly 56 and a controller 36. The controller 36 is operative to acquire MR data 74 from the object 84 via the magnet assembly 56. At least two portions 94, 96, 98 of the MR data 74 are acquired with different readout gradient amplitudes 88, 90, 92 of the plurality.

FIG. 1

EP 3 480 615 A1

## Description

Embodiments of the invention relate generally to magnetic resonance imaging ("MRI") systems, and more specifically, to a system and method for magnetic resonance imaging an object with a plurality of readout gradient amplitudes.

MRI is a widely accepted and commercially available technique for obtaining digitized visual images representing the internal structure of objects having substantial populations of atomic nuclei that are susceptible to nuclear magnetic resonance ("NMR"). Many MRI systems use superconductive magnets to scan a subject/patient via imposing a strong main magnetic field on the nuclei in the subject to be imaged. The nuclei are excited by a radio frequency ("RF") signal/pulse transmitted by a RF coil at characteristics NMR (Larmor) frequencies. By spatially disturbing localized magnetic fields surrounding the subject and analyzing the resulting RF responses, also referred to hereinafter as the "MR signal," from the nuclei as the excited protons relax back to their lower energy normal state, a map or image of these nuclei responses as a function of their spatial location is generated and displayed. An image of the nuclei responses, also referred to hereinafter as an "MRI image," provides a non-invasive view of a subject's internal structure.

The delay between excitation of the nuclei by a RF pulse, e.g., a 90° RF pulse, and the sampling of the MR signal following a free-induction decay ("FID"), gradient echo, or spin echo is known as the "echo time" and/or "TE". The amount of time required for an MRI system to obtain/read MR data for a given portion of K-Space is commonly known as a "readout duration." In order to image species of nuclei having short transverse relaxation times, ("T2"), some MRI systems, commonly referred to as "Zero TE" MRI systems and/or "Ultra Short TE ('UTE')" MRI systems, sample the MR signal at zero and/or near zero delay/TE after a RF pulse.

Low imaging bandwidth and/or high spatial resolution in such UTE or Zero TE MRI systems, however, may result in readout durations similar to the fat-water in-phase echo time, e.g., 2.3 ms at 3T, which in turn may result in the occurrence of fat-water interferences. Such interferences often disturb soft-tissue uniformity, thus making it difficult to distinguish air, soft tissue, and bone. Increasing the imaging bandwidth, or lowering the spatial resolution may reduce the readout duration, which in turn may reduce fat-water interference. MRI images having low spatial resolution, however, may be unsatisfactory for certain medical diagnostics. On the other hand, the imaging Bandwidth of Zero TE imaging is typically limited by hardware constraints.

What is needed, therefore, is an improved system and method for magnetic resonance imaging an object with a plurality of readout gradient amplitudes.

In an embodiment, a system for magnetic resonance imaging an object with a plurality of readout gradient amplitudes is provided. The system includes a magnet assembly and a controller. The controller is operative to acquire MR data from the object via the magnet assembly. At least two portions of the MR data are acquired with different readout gradient amplitudes of the plurality.

In another embodiment, a method of magnetic resonance imaging an object with a plurality of readout gradient amplitudes is provided. The method includes acquiring MR data from the object via a MRI system. At least two portions of the MR data are acquired with different readout gradient amplitudes of the plurality.

In yet another embodiment, a non-transitory computer readable medium including instructions is provided. The instructions are configured to adapt a controller to acquire MR data from an object via a magnet assembly. At least two portions of the MR data are acquired with different readout gradient amplitudes.

The present invention will be better understood from reading the following description of non-limiting embodiments, with reference to the attached drawings, wherein below:

FIG. 1 is a block diagram of an exemplary system for magnetic resonance imaging an object with a plurality of readout gradient amplitudes, in accordance with an embodiment of the present invention;

FIG. 2 is a schematic cross-sectional diagram of a magnet assembly of the system of FIG. 1, in accordance with an embodiment of the present invention;

FIG. 3 is a flow chart depicting a method for magnetic resonance imaging an object with a plurality of readout gradient amplitudes utilizing the system of FIG. 1, in accordance with an embodiment of the present invention;

FIG. 4 is graphical chart depicting the plurality of readout gradient amplitudes of FIG. 1, in accordance with an embodiment of the present invention;

FIG. 5 is a diagram of a K-Space acquired by the system of FIG. 1, in accordance with an embodiment of the present invention;

FIG. 6 is a diagram of a plurality of image sets generated by the system of FIG. 1, in accordance with an embodiment of the present invention; and

FIG. 7 is a diagram of an image generated based at least in part on an in-phase component of MR data acquired by the system of FIG. 1, in accordance with an embodiment of the present invention.

Reference will be made below in detail to exemplary embodiments of the invention, examples of which are illustrated in the accompanying drawings. Wherever possible, the same reference characters used throughout the drawings refer to the same or like parts, without duplicative description.

As used herein, the terms "substantially," "generally," and "about" indicate conditions within reasonably achievable manufacturing and assembly tolerances, relative to ideal desired conditions suitable for achieving the functional purpose of a component or assembly. As used herein, "electrically coupled," "electrically connected," and "electrical communication" mean that the referenced elements are directly or indirectly connected such that an electrical current may flow from one to the other. The connection may include a direct conductive connection, i.e., without an intervening capacitive, inductive or active element, an inductive connection, a capacitive connection, and/or any other suitable electrical connection. Intervening components may be present. The term "real-time," as used herein, means a level of processing responsiveness that a user senses as sufficiently immediate or that enables the processor to keep up with an external process. The term "MR data," as used herein, refers to data, e.g., raw K-Space and/or image space, derived from an MR signal. The terms "readout gradient amplitude" and "$G_{read}$" refer to the magnetic field gradient applied during the time period when an MRI system is reading/sampling a MR signal emitted from an object. The term "in-phase," as used herein with respect to the component of an MR signal and/or MR data, refers to component of the MR signal and/or MR data generated from in-phase water and fat contributions, e.g., zero relative phase difference between fat and water. Similarly, the term "out-of-phase," as used herein with respect to the component of an MR signal and/or MR data, refers to the component of the MR signal and/or MR data generated from out-of-phase water and fat contributions, e.g., 180° relative phase difference between fat and water).

Further, while the embodiments disclosed herein are described with respect to a Zero TE MRI system, it is to be understood that embodiments of the present invention may be applicable to other imaging systems to include UTE MRI and/or any type of MRI based imaging system. Additionally, embodiments of the present invention may also be combined with more traditional echo-time shifting methods used for fat-water separation. Further still, as will be appreciated, embodiments of the present invention related imaging systems may be used to analyze tissue generally and are not limited to human tissue.

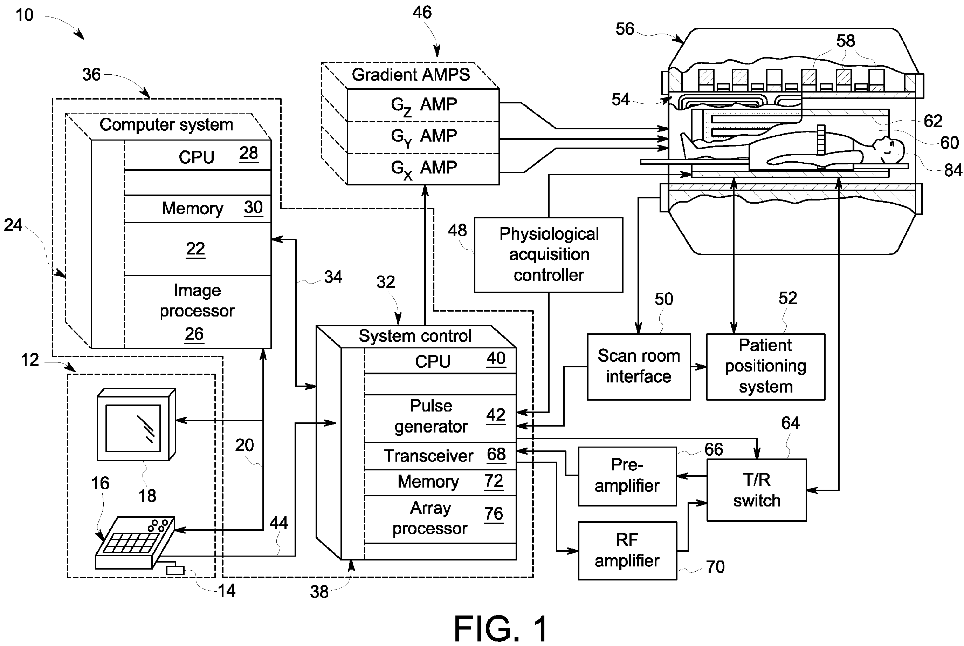

Accordingly, referring now to FIG. 1, the major components of an MRI system 10 incorporating an embodiment of the invention are shown. Operation of the system 10 is controlled from the operator console 12, which includes a keyboard or other input device 14, a control panel 16, and a display screen 18. The console 12 communicates through a link 20 with a separate computer system 22 that enables an operator to control the production and display of images on the display screen 18. The computer system 22 includes a number of modules, which communicate with each other through a backplane 24. These include an image processor module 26, a CPU module 28 and a memory module 30, which may include a frame buffer for storing image data arrays. The computer system 22 communicates with a separate system control or control unit 32 through a high-speed serial link 34. The input device 14 can include a mouse, joystick, keyboard, track ball, touch activated screen, light wand, voice control, or any similar or equivalent input device, and may be used for interactive geometry prescription. The computer system 22 and the MRI system control 32 collectively form an "MRI controller" 36.

The MRI system control 32 includes a set of modules connected together by a backplane 38. These include a CPU module 40 and a pulse generator module 42, which connects to the operator console 12 through a serial link 44. It is through link 44 that the system control 32 receives commands from the operator to indicate the scan sequence that is to be performed. The pulse generator module 42 operates the system components to execute the desired scan sequence and produces data which indicates the timing, strength and shape of the RF pulses produced, and the timing and length of the data acquisition window. The pulse generator module 42 connects to a set of gradient amplifiers 46, to indicate the timing and shape of the gradient pulses that are produced during the scan. The pulse generator module 42 can also receive patient data from a physiological acquisition controller 48 that receives signals from a number of different sensors connected to the patient, such as ECG signals from electrodes attached to the patient. And finally, the pulse generator module 42 connects to a scan room interface circuit 50, which receives signals from various sensors associated with the condition of the patient and the magnet system. It is also through the scan room interface circuit 50 that a patient positioning system 52 receives commands to move the patient to the desired position for the scan.

The pulse generator module 42 operates the gradient amplifiers 46 to achieve desired timing and shape of the gradient pulses that are produced during the scan. The gradient waveforms produced by the pulse generator module 42 are applied to the gradient amplifier system 46 having Gx, Gy, and Gz amplifiers. Each gradient amplifier excites a corresponding physical gradient coil in a gradient coil assembly, generally designated 54, to produce the magnetic field gradients used for spatially encoding acquired signals. The gradient coil assembly 54 forms part of a magnet assembly 56, which also includes a polarizing magnet 58 (which in operation, provides a homogenous longitudinal magnetic field Bo throughout a target volume 60 that is enclosed by the magnet assembly 56) and a whole-body (transmit and receive) RF coil 62 (which, in operation, provides a transverse magnetic field $B_1$ that is generally perpendicular to Bo throughout the target volume 60).

The resulting signals emitted by the excited nuclei in the patient may be sensed by the same RF coil 62 and coupled through the transmit/receive switch 64 to a preamplifier 66. The amplifier MR signals are demodulated, filtered, and digitized in the receiver section of a transceiver 68. The transmit/receive switch 64 is controlled by a signal from the pulse generator module 42 to electrically connect an RF amplifier 70 to the RF coil 62 during the transmit mode and to connect the preamplifier 66 to the RF coil 62 during the receive mode. The transmit/receive switch 64 can also enable a separate RF coil (for example, a surface coil) to be used in either transmit or receive mode.

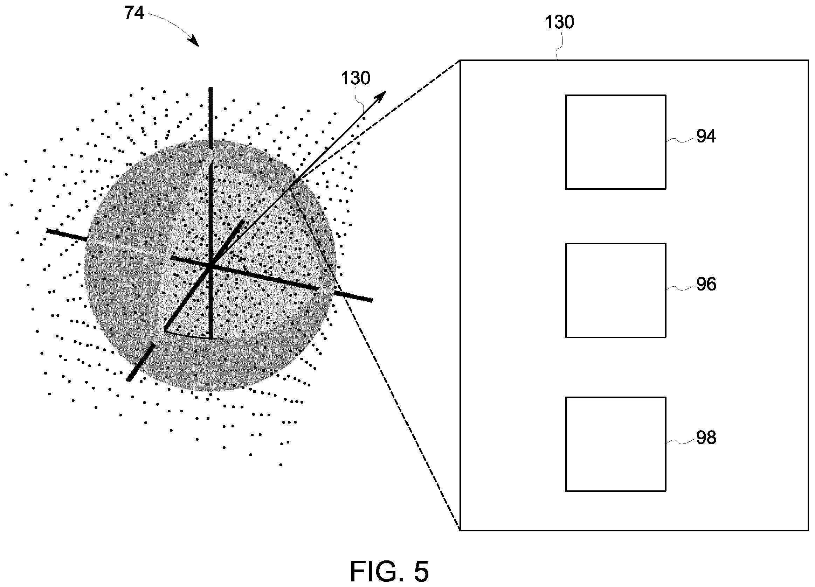

The MR signals picked up by the RF coil 62 are digitized by the transceiver module 68 and transferred to a memory module 72 in the system control 32. A scan is complete when an array of raw K-Space data 74 (FIG. 5) has been acquired in the memory module 72. This raw K-Space data/datum is rearranged into separate K-Space data arrays for each image to be reconstructed, and each of these is input to an array processor 76 which operates to Fourier transform the data into an array of image data. This image data is conveyed through the serial link 34 to the computer system 22 where it is stored in memory 30. In response to commands received from the operator console 12, this image data may be archived in long-term storage or it may be further processed by the image processor 26 and conveyed to the operator console 12 and presented on the display 18.

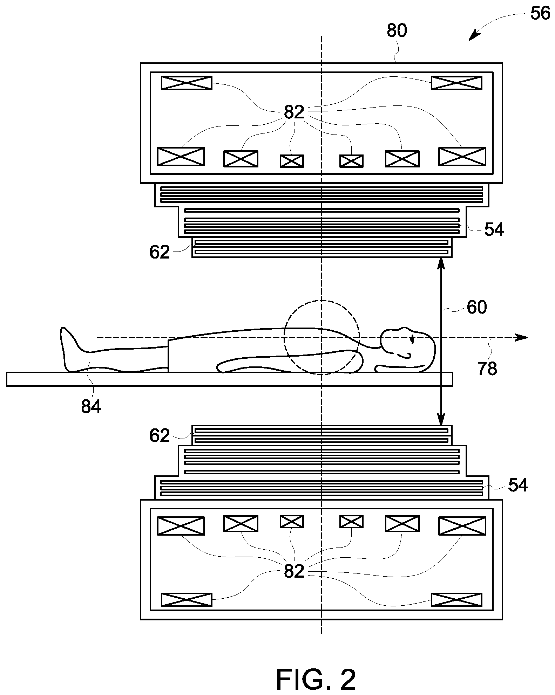

As illustrated in FIG. 2, a schematic side elevation view of the magnet assembly 56 is shown in accordance with an embodiment of the invention. The magnet assembly 56 is cylindrical in shape having a center axis 78. The magnet assembly 56 includes a cryostat 80 and one or more radially aligned longitudinally spaced apart superconductive coils 82 that form the polarizing magnet 58 (FIG. 1). The superconductive coils 82 are capable of carrying large electrical currents and are designed to create the Bo field within the patient/target volume 60. As will be appreciated, the magnet assembly 56 may further include both a terminal shield and a vacuum vessel (not shown) surrounding the cryostat 80 in order to help insulate the cryostat 80 from heat generated by the rest of the MRI system 10 (FIG. 1). The magnet assembly 56 may still further include other elements such as covers, supports, suspension members, end caps, brackets, etc. (not shown). While the embodiment of the magnet assembly 56 shown in FIGS. 1 and 2 utilizes a cylindrical topology, it should be understood that topologies other than cylindrical may be used. For example, a flat geometry in a split-open MRI system may also utilize embodiments of the invention described below. As further shown in FIG. 2, a patient / imaged subject 84 is inserted into the magnet assembly 56.

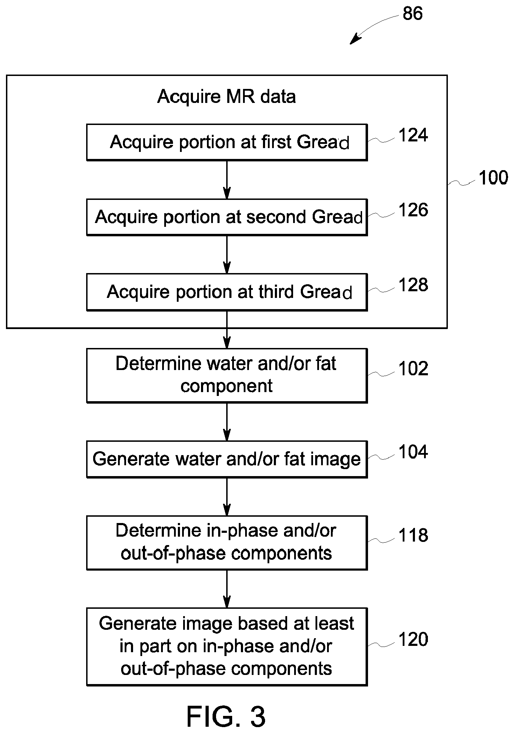

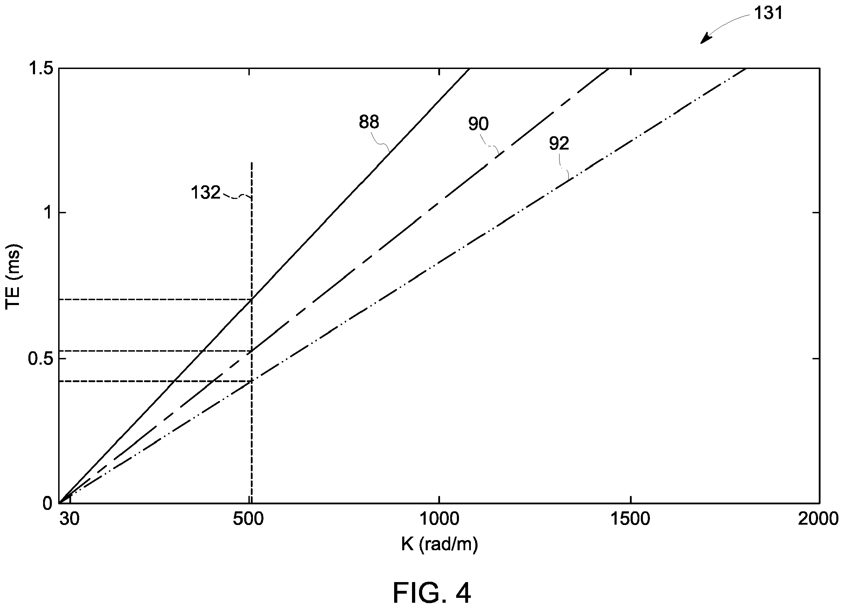

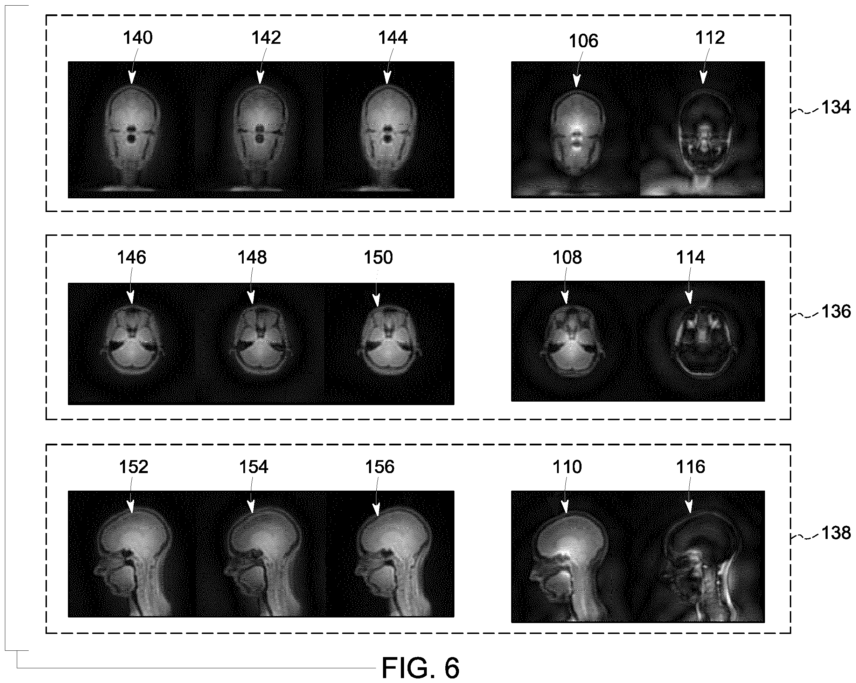

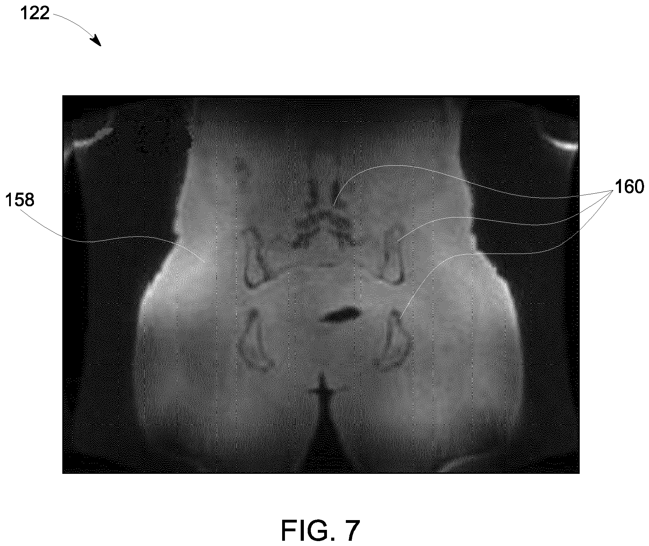

Turning now to FIG. 3, a method 86 for magnetic resonance imaging the patient/object/subject 84 (FIGS. 1 and 2) with a plurality of readout gradient amplitudes/$G_{reads}$ 88, 90, 92 (FIG. 4) is shown. As will be explained in greater detail below, in embodiments, the method 86 includes acquiring 100 MR data 74 (FIG. 5) from the object 84 via the MRI system 10 with at least two portions 94, 96, 98 (FIG. 5) of the MR data 74 acquired with different $G_{reads}$ of the plurality 88, 90, 92. The method 86 may also include determining 102 at least one of a water component and a fat component of the MR signal/data 74 based at least in part on the at least two portions 94, 96, 98 of the MR data 74. In certain aspects, the MRI controller 36 may generate 104 at least one of a water image 106, 108, and 110 (FIG. 6) and a fat image 112, 114, 116 (FIG. 6) based at least in part on the at least two portions 94, 96, 98 of the MR data 74. The method 86 may also include determining 118 at least one of an in-phase component and an out-of-phase component of the MR data 74 based at least in part on the at least two portions 94, 96, 98 of the MR data 74. As such, the method 86 may further include generating 120 an image 122 (FIG. 7) based at least in part on the in-phase component of the MR data 74.

Accordingly, as shown in FIG. 3, acquiring 100 the MR data 74 may include acquiring 124, 126, 128 the at least two portions 94, 96, 98 (FIG. 5) sequentially such that each of the at least two portions 94, 96, 98 corresponds to a same segment 130 (FIG. 5) of the MR data 74, e.g., the same radial within K-Space. In other words, the at least two portions 94, 96, 98 may be acquired 124, 126, 128 by sampling/reading the same segment 130 of K-Space 74, with each acquisition 124, 126, 128 utilizing a different $G_{read}$ 88, 90, 92 (FIG. 4). Thus, each portion 94, 96, 98 may contain different MR data values for the same K-Space locations. As will be appreciated, sequentially acquiring the portions 94, 96, 98 over the same segment 130 may serve to reduce motion artifacts.

Referring now to FIG. 4, a graphical chart 131 depicting the at least two $G_{reads}$ 88, 90, 92 is shown where the vertical axis represents TE in ms and the longitudinal axis represents K-Space distances in rad/m. As will be appreciated, the higher the $G_{read}$ of a readout/acquisition 124, 126, 128 (FIG. 3), the shorter the TE for the readout/acquisition 124, 126, 128, and thus, the more K-Space locations which can be traversed/read/sampled/obtained during the acquisition 124, 126, 128. In embodiments, each $G_{read}$ 88, 90, 92 may be determined by the imaging bandwidth ("BW") and the field-of-view ("FOV") according to BW = $\gamma$*Gread*FOV, and may be limited by physiological and hardware gradient constraints typically to below one-hundred (100) mT/m.

Accordingly, a first $G_{read}$ 90 having a higher value than a second $G_{read}$ 88 may reach the same K-Space 74 location (represented by vertical line 132) sooner than the second $G_{read}$ 88. In other words, different $G_{reads}$ result in different readout speeds such that a K-Space location, e.g., location 132, may be encoded at different TEs. For example, as shown in FIG. 4, a $G_{read}$ 88 of about 3.6 mT/m (characteristic for a FOV=0.4 m and a BW=±31 kHz) may reach location 132 (k = 500 rad/m) at about 0.51 s. Decreasing or Increasing the gradient readout amplitude by 25% (Gread = 2.7 mT/m, Gread =4.5 mT/m), correspondingly increases, or decreases the time required to read location 132 k=500 rad/s (to 0.68 s and 0.41 s, respectively). As will be appreciated, the chemical shift fat-water contrast between the value, i.e., the MR signal, of a particular K-Space location over multiple acquisitions, i.e., across the portions 94, 96, 98, increases towards the outer edges of the K-Space, i.e., towards the right side of the chart 131. In other words, the father away from central K-Space (represented by the origin of chart 131) a particular location is, the greater the difference in TE and the corresponding MR signal value of the location across the portions 94, 96, and 98. Thus, by reading the same K-Space 74 segment 130 with different $G_{reads}$, a system of equations/model may be used to individually solve for the water and/or fat components, and/or the in-phase and/or out-of-phase components, of the MR signal/data 74.

For example, in embodiments, the MR signal may be represented by the following equation:

$$data(k = \gamma\, G_{read}\, TE_n) = \int d^3 r\, \left[image(r, TE_n)\, e^{i\,\gamma\, G_{read}\, TE_n\, r}\right]$$

where $\gamma$ is the gyromagnetic ratio, and where $G_{read}$ is the readout gradient strengths determined by the BW and FOV according to BW = $\gamma$*Gread*FOV. The effective echo time TEn describes the time needed to reach a certain k-space location kn = $\gamma$*Gread*TEn and is determined by the sampling time $\Delta t$ = 1/BW according to Ten = n*$\Delta t$. As will be understood, the argument in the exponential function describes the usual K-Space 74 encoding, i.e., exp(i kn r) = exp(i $\gamma$ $G_{read}$ TEn r). Thus, by decomposing the image into image(r,TEn) = wat(r)*exp(i $\omega_{wat}$ TE$_n$) + fat(r)*exp(i $\omega_{fat}$ TE$_n$), with $\omega_{water}$ and $\omega_{fat}$ (the water and fat chemical shift frequencies), the above equation can be restated as:

$$data(k_n = \gamma\, G_{read}\, TE_n)$$

$$= \int d^3 r\, \left[wat(r, TE_n) e^{i\,\omega_{water}\, TE_n} + fat(r, TE_n) e^{i\,\omega_{fat}\, TE_n}\right] e^{i\,\gamma\, G_{read}\, TE_n\, r}$$

$$= e^{i\,\omega_{wat}\, TE_n}\, data_{wat}(\gamma\, G_m\, TE_n) + e^{i\,\omega_{fat}\, TE_n}\, data_{fat}(\gamma\, G_{read}\, TE_n)$$

which, as will be appreciated, may be used for K-Space water-fat decomposition.

For example, in a scenario where three Zero TE acquisitions are identical except for their Gread, which may be multiplied by $\alpha$ for the second experiment, and by $\beta$ for the third experiment, e.g., {Gread, $\alpha$*Gread, $\beta$*Gread}, a fixed K-Space location 132 may have the following overdetermined linear system of equations (three equations for two unknowns):

$$\begin{bmatrix} data_1(k = \gamma\, 1 * G_{read}\, TE_n/1) \\ data_2(k = \gamma\, \alpha * G_{read}\, TE_n/\alpha) \\ data_3(k = \gamma\, \beta * G_{read}\, TE_n/\beta) \end{bmatrix}$$

$$= \begin{bmatrix} e^{i\,\omega_{wat}\, TE_n/1} & e^{i\,\omega_{fat}\, TE_n/1} \\ e^{i\,\omega_{wat}\, TE_n/\alpha} & e^{i\,\omega_{fat}\, TE_n/\alpha} \\ e^{i\,\omega_{wat}\, TE_n/\beta} & e^{i\,\omega_{fat}\, TE_n/\beta} \end{bmatrix} * \begin{bmatrix} data_{wat}(\gamma\, G_{read}\, TE_n) \\ data_{fat}(\gamma\, G_{read}\, TE_n) \end{bmatrix}$$

which may be solved for $data_{wat}$ and $data_{fat}$ for each k via simple (regularized) inversion of the chemical shift encoding matrix. As will be appreciated, in embodiments, that fat-water decomposition may be achieved due to the same nominal K-Space position k = $\gamma G_{read}$ TE$_n$ being acquired at different TEs = {TE$_n$, TE$_{n/\alpha}$, TE$_n/\beta$} for readout gradient strengths of {$G_{read}$, $G_{read}/\alpha$, $G_{read}/\beta$}. Accordingly, in certain aspects, the ability for fat water decomposition depends on the conditioning of the chemical shift matrix in the above equation, and breaks down in cases where the echo time spacing $\Delta TE_n$ approaches multiples e.g., {0,1,2, ...}, of the fat-water precession time, e.g., 1/440Hz = 2.3 ms at 3T.

Alternatively, the data can also be decomposed into in-phase (dIP = dwat + dfat) and out-phase (dOP = dwat - dfat) contributions and corresponding images. After fat-water, or in-phase-out-phase decomposition, the data are reconstructed into corresponding images using existing non-Cartesian image reconstruction methods using optional parallel imaging, compressed sensing, or regularization methods.

Accordingly, referring now to FIG. 6, a series of three different image sets 134, 136, and 138 respectively depicting coronal, middle, and sagittal slices of a patient's 84 head are shown. As will be appreciated, the image sets 134, 136, and 138 shown in FIG. 6 were acquired via a Zero TE fat-water separation experiment, performed in accordance with an embodiment of the present invention, with a Transmit/Receive body coil having a FOV = 40 cm, a BW = $\pm$31.25 kHz, and a FA = 1. As will be understood, the typical readout gradient strength of Gread = 3.67 mT/m, often used for Zero TE experiments, was changed to: Gread = 3.6 mT/m for the left column images 140, 146, and 152; Gread = 4.5 mT/m for the 2nd column images 142, 148, and 154; and Gread = 5.4 mT/m for the third column images 144, 150, and 156. Using an embodiment of the above described method, corresponding water (the 4th column 106, 108, and 110) and fat (the 5th column 112, 114, and 116) images were reconstructed.

In other words, each image set 134, 136, and 138 includes three images representative of the portions 94, 96, 98 acquired 124, 126, 128 at three different $G_{reads}$, as well as a water image and a fat image, were the water image and the fat image are generated by solving for the water and fat components of the MR signal based on the MR data 74 within the portions 94, 96, 98 in accordance with the above-described equations. For example, image set 134 includes three images 140, 142, and 144 each acquired with different $G_{reads}$ 88, 90, and 92, respectively, and water image 106 and fat image 112. As will be appreciated, water 106 and fat image 112 may be generated by solving for the water and fat components of the MR data 74 within the three images 140, 142, and 144. Similarly, image set 136 includes three images 146, 148, and 150 each acquired with different $G_{reads}$ 88, 90, and 92, respectively, and water image 108 and fat image 114; and image set 138 includes three images 152, 154, and 156 each acquired with different $G_{reads}$ 88, 90, and 92, respectively, and water image 110 and fat image 116.

Turning to FIG. 7, as previously discussed, the above equations may also provide for the ability to solve for the in-phase and/or out-of-phase components of the MR signal/data 74, which in turn, may be used to generate 120 an image 122. As will be appreciated, generating 120 an image 122 based at least in part on the in-phase component of the MR signal/data 74 may improve the uniformity of the MR signal/data 74 corresponding to soft tissue 158, which in turn reduces the effects of fat-water interference so that visualization of bone structures 160 is improved. For example, embodiments of the present invention may generate three image sets, similar to image sets 134, 136, and 138 shown in FIG. 6, wherein the image sets respectively represent a coronal, a sagittal, and an axial slice of an object, e.g., a human pelvis, acquired via a Zero TE in-phase-out-phase separation experiment by a surface coil array having a FOV = 32 cm, a BW = $\pm$31.25kHz, and a FA=1. As will be understood, the typical readout gradient strength of Gread = 4.5874 mT/m, often used for Zero TE experiments, may be changed to Gread = 4.6mT/m for the first image of each set, a Gread = 3.54 mT/m for the second image in each set, and a Gread = 5.75 mT/m for the third image in each set. Using the above described in-phase-out-phase separation method, corresponding in-phase images demonstrating improved soft-tissue uniformity clean of destructive fat-water interferences, which is often advantageous for bone imaging and segmentation. As will be appreciated, the level of soft-tissue uniformity provided by embodiments of the present invention, as shown in image 122, is difficult, and often non-feasible, to obtain using standard MR methods.

While the embodiments herein depict three portions 94, 96, and 98 acquired by three acquisitions 124, 126, and 128, with three readout gradient amplitudes 88, 90, and 92, it will be understood that other embodiments may use as few as two portions 94 and 96 acquired by two acquisitions 124 and 126, with two readout gradient amplitudes 88 and 90. Yet other embodiments may use four or more portions acquired by four or more acquisitions, with four or more readout gradient amplitudes. Additionally, the gradient amplitudes may be determined by the FOV and BW such that they typically cover the full range allowed by the gradient hardware and/or physiological constraints. Accordingly, in embodiments, each readout gradient amplitude of the plurality may be scaled by a factor of about 0.3 to about 3.0, and in some embodiments, of about .75 to about 1.25. As will be appreciated, however, other embodiments may have different scaling factors.

Finally, it is also to be understood that the system 10 may include the necessary electronics, software, memory, storage, databases, firmware, logic/state machines, microprocessors, communication links, displays or other visual or audio user interfaces, printing devices, and any other input/output interfaces to perform the functions described herein and/or to achieve the results described herein. For example, as previously mentioned, the system may include at least one processor and system memory / data storage structures, which may include random access memory (RAM) and read-only memory (ROM). The at least one processor of the system 10 may include one or more conventional microprocessors and one or more supplementary co-processors such as math co-processors or the like. The data storage structures discussed herein may include an appropriate combination of magnetic, optical and/or semiconductor memory, and may include, for example, RAM, ROM, flash drive, an optical disc such as a compact disc and/or a hard disk or drive.

Additionally, a software application that adapts the controller to perform the methods disclosed herein may be read into a main memory of the at least one processor from a computer-readable medium. The term "computer-readable medium", as used herein, refers to any medium that provides or participates in providing instructions to the at least one processor of the system 10 (or any other processor of a device described herein) for execution. Such a medium may take many forms, including but not limited to, non-volatile media and volatile media. Non-volatile media include, for example, optical, magnetic, or opto-magnetic disks, such as memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, a RAM, a PROM, an EPROM or EEPROM (electronically erasable programmable read-only memory), a FLASH-EEPROM, any other memory chip or cartridge, or any other medium from which a computer can read.

While in embodiments, the execution of sequences of instructions in the software application causes at least one processor to perform the methods/processes described herein, hard-wired circuitry may be used in place of, or in combination with, software instructions for implementation of the methods/processes of the present invention. Therefore, embodiments of the present invention are not limited to any specific combination of hardware and/or software.

It is further to be understood that the above description is intended to be illustrative, and not restrictive. For example, the above-described embodiments (and/or aspects thereof) may be used in combination with each other. Additionally, many modifications may be made to adapt a particular situation or material to the teachings of the invention without departing from its scope.

For example, in an embodiment, a system for magnetic resonance imaging an object with a plurality of readout gradient amplitudes is provided. The system includes a magnet assembly and a controller. The controller is operative to acquire MR data from the object via the magnet assembly. At least two portions of the MR data are acquired with different readout gradient amplitudes of the plurality. In certain embodiments, the controller is further operative to determine at least one of a water component and a fat component of the MR data based at least in part on the at least two portions of the MR data. In certain embodiments, the controller is further operative to generate at least one of a water image and a fat image based at least in part on at least one of the water component and the fat component. In certain embodiments, the controller is further operative to determine at least one of an in-phase component and an out-of-phase component of the MR data based at least in part on the at least two portions of the MR data. In certain embodiments, the controller is further operative to generate an image based at least in part on the in-phase component of the MR data. In certain embodiments, each readout gradient amplitude of the plurality is scaled by a factor of about 0.3 to about 3.0. In certain embodiments, the controller acquires the MR data based at least in part on Zero TE. In certain embodiments, the at least two portions correspond to a same segment of the MR data.

Yet other embodiments provide for a method of magnetic resonance imaging an object with a plurality of readout gradient amplitudes. The method includes acquiring MR data from the object via a MRI system. At least two portions of the MR data are acquired with different readout gradient amplitudes of the plurality. In certain embodiments, the method further includes determining at least one of a water component and a fat component of the MR data based at least in part on the at least two portions of the MR data. In certain embodiments, the method further includes generating at least one of a water image and a fat image based at least in part on at least one of the water component and the fat component. In certain embodiments, the method further includes determining at least one of an in-phase component and an out-of-phase component of the MR data based at least in part on the at least two portions of the MR data. In certain embodiments, the method further includes generating an image based at least in part on the in-phase component of the MR data. In certain embodiments, each readout gradient amplitude of the plurality is scaled by a factor of about 0.3 to about 3.0. In certain embodiments, the controller acquires the MR data based at least in part on Zero TE. In certain embodiments, the at least two portions correspond to a same segment of the MR data.

Yet still other embodiments provide for a non-transitory computer readable medium that includes instructions. The instructions are configured to adapt a controller to acquire MR data from an object via a magnet assembly. At least two portions of the MR data are acquired with different readout gradient amplitudes. In certain embodiments, the instructions are further configured to adapt the controller to determine at least one of a water component and a fat component of the MR data based at least in part on the at least two portions of the MR data. In certain embodiments, the instructions are further configured to adapt the controller to determine at least one of an in-phase component and an out-of-phase component of the MR data based at least in part on the at least two portions of the MR data. In certain embodiments, the controller acquires the MR data based at least in part on Zero TE.

Accordingly, by obtaining multiple acquisitions of the same segment of K-Space with each acquisition using a different $G_{read}$, some embodiments of the present invention may provide for Zero TE MR imaging of an object clean of fat-water interference effects. In other words, some embodiments of the present invention provide for the ability to acquire a MR data set which can be fitted to a system of equations that provides for the ability to solve for the water and/or fat components, as well as the in-phase and/or out-of-phase components, of an acquired MR signal/data in Zero TE MRI systems. Thus, some embodiments of the present invention may mitigate the effects of fat-water interference without requiring the use of high imaging bandwidths. As such, some embodiments of the present invention provide for a method of mitigating fat-water interference in a wide range of Zero TE MRI systems, some of which may not have hardware components capable of achieving high imaging bandwidths. Thus, some embodiments provide for improved bone segmentation for Zero TE MRI systems.

Additionally, while the dimensions and types of materials described herein are intended to define the parameters of the invention, they are by no means limiting and are exemplary embodiments. Many other embodiments will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, terms such as "first," "second," "third," "upper," "lower," "bottom," "top," etc. are used merely as labels, and are not intended to impose numerical or positional requirements on their objects. Further, the limitations of the following claims are not written in means-plus-function format are not intended to be interpreted as such, unless and until such claim limitations expressly use the phrase "means for" followed by a statement of function void of further structure.

This written description uses examples to disclose several embodiments of the invention, including the preferred mode, and also to enable one of ordinary skill in the art to practice the embodiments of invention, including making and using any devices or systems and performing any incorporated methods. The patentable scope of the invention is defined by the claims, and may include other examples that occur to one of ordinary skill in the art. Such other examples are intended to be within the scope of the claims if they have structural elements that do not differ from the literal language of the claims, or if they include equivalent structural elements with insubstantial differences from the literal languages of the claims.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property.

Since certain changes may be made in the above-described invention, without departing from the spirit and scope of the invention herein involved, it is intended that all of the subject matter of the above description shown in the accompanying drawings shall be interpreted merely as examples illustrating the inventive concept herein and shall not be construed as limiting the invention.

Various aspects and embodiments of the present invention are defined by the following numbered clauses:

1. A system for magnetic resonance imaging an object with a plurality of readout gradient amplitudes, the system comprising:

   a magnet assembly; and
   a controller operative to:

      acquire MR data from the object via the magnet assembly,

      wherein at least two portions of the MR data are acquired with different readout gradient amplitudes of the plurality.

2. The system of clause 1, wherein the controller is further operative to:

   determine at least one of a water component and a fat component of the MR data based at least in part on the at least two portions of the MR data.

3. The system of any preceding clause, wherein the controller is further operative to:

   generate at least one of a water image and a fat image based at least in part on at least one of the water component and the fat component.

4. The system of any preceding clause, wherein the controller is further operative to:

   determine at least one of an in-phase component and an out-of-phase component of the MR data based at least in part on the at least two portions of the MR data.

5. The system of any preceding clause, wherein the controller is further operative to:

   generate an image based at least in part on the in-phase component of the MR data.

6. The system of any preceding clause, wherein each readout gradient amplitude of the plurality is scaled by a factor of about 0.3 to about 3.0.

7. The system of any preceding clause, wherein the controller acquires the MR data based at least in part on Zero TE.

8. The system of any preceding clause, wherein the at least two portions correspond to a same segment of the MR data.

9. A method of magnetic resonance imaging an object with a plurality of readout gradient amplitudes, the method comprising:

acquiring MR data from the object via a MRI system,
wherein at least two portions of the MR data are acquired with different readout gradient amplitudes of the plurality.

10. The method of any preceding clause further comprising:

determining at least one of a water component and a fat component of the MR data based at least in part on the at least two portions of the MR data.

11. The method of any preceding clause further comprising:

generating at least one of a water image and a fat image based at least in part on at least one of the water component and the fat component.

12. The method of any preceding clause further comprising:

determining at least one of an in-phase component and an out-of-phase component of the MR data based at least in part on the at least two portions of the MR data.

13. The method of any preceding clause further comprising:

generating an image based at least in part on the in-phase component of the MR data.

14. The method of any preceding clause, wherein each readout gradient amplitude of the plurality is scaled by a factor of about 0.3 to about 3.0.

15. The method of any preceding clause, wherein the controller acquires the MR data based at least in part on Zero TE.

16. The method of any preceding clause, wherein the at least two portions correspond to a same segment of the MR data.

17. A non-transitory computer readable medium comprising instructions configured to adapt a controller to:

acquire MR data from an object via a magnet assembly,
wherein at least two portions of the MR data are acquired with different readout gradient amplitudes.

18. The non-transitory computer readable medium of any preceding clause, wherein the instructions are further configured to adapt the controller to:

determine at least one of a water component and a fat component of the MR data based at least in part on the at least two portions of the MR data.

19. The non-transitory computer readable medium of any preceding clause, wherein the instructions are further configured to adapt the controller to:

determine at least one of an in-phase component and an out-of-phase component of the MR data based at least in part on the at least two portions of the MR data.

20. The non-transitory computer readable medium of any preceding clause, wherein the controller acquires the MR data based at least in part on Zero TE.

**Claims**

1.  A system (10) for magnetic resonance imaging an object (84) with a plurality of readout gradient amplitudes (88), (90), (92), the system (10) comprising:

    a magnet assembly (56); and
    a controller (36) operative to:

    acquire MR data (74) from the object (84) via the magnet assembly (56),

    wherein at least two portions (94), (96), (98) of the MR data (74) are acquired with different readout gradient amplitudes (88), (90), (92) of the plurality.

2.  The system (10) of claim 1, wherein the controller (36) is further operative to:

    determine at least one of a water component and a fat component of the MR data (74) based at least in part on the at least two portions (94), (96), (98) of the MR data (74).

3.  The system (10) of claim 2, wherein the controller (36) is further operative to:

    generate at least one of a water image (106) and a fat image (112) based at least in part on at least one of the water component and the fat component.

4.  The system (10) of any preceding claim, wherein the controller (36) is further operative to:

    determine at least one of an in-phase component and an out-of-phase component of the MR data (74) based at least in part on the at least two portions (94), (96), (98) of the MR data (74).

5.  The system (10) of claim 4, wherein the controller (36) is further operative to:

    generate an image (122) based at least in part on the in-phase component of the MR data (74).

6.  The system (10) of any preceding claim, wherein each readout gradient amplitude (88), (90), (92) of the plurality is scaled by a factor of about 0.3 to about 3.0.

7.  The system (10) of any preceding claim, wherein the controller (36) acquires the MR data (74) based at least in part on Zero TE.

8.  The system (10) of claim 7, wherein the at least two portions (94), (96), (98) correspond to a same segment (130) of the MR data (74).

9.  A method (86) of magnetic resonance imaging an object (84) with a plurality of readout gradient amplitudes (88), (90), (92), the method (86) comprising:

    acquiring (100) MR data (74) from the object (84) via a MRI system (10),
    wherein at least two portions (94), (96), (98) of the MR data (74) are acquired with different readout gradient amplitudes (88), (90), (92) of the plurality.

10. The method (86) of claim 9 further comprising:

    determining (102) at least one of a water component and a fat component of the MR data (74) based at least in part on the at least two portions (94), (96), (98) of the MR data (74).

11. The method (86) of claim 10 further comprising:

    generating (104) at least one of a water image (106) and a fat image (112) based at least in part on at least one of the water component and the fat component.

**12.** The method 86 of any of claims 9 to 11 further comprising:

determining (118) at least one of an in-phase component and an out-of-phase component of the MR data (74) based at least in part on the at least two portions (94), (96), (98) of the MR data (74).

**13.** The method (86) of claim 12 further comprising:

generating (120) an image (122) based at least in part on the in-phase component of the MR data (74).

**14.** The method (86) of any of claims 9 to 13, wherein each readout gradient amplitude (88), (90), (92) of the plurality is scaled by a factor of about 0.3 to about 3.0.

**15.** The method (86) of any of claims 9 to 14, wherein the the MR data (74) is acquired based at least in part on Zero TE.

FIG. 1

FIG. 2

86

Acquire MR data

Acquire portion at first Gread — 124

Acquire portion at second Gread — 126

Acquire portion at third Gread — 128

100

Determine water and/or fat component — 102

Generate water and/or fat image — 104

Determine in-phase and/or out-of-phase components — 118

Generate image based at least in part on in-phase and/or out-of-phase components — 120

FIG. 3

FIG. 4

EP 3 480 615 A1

FIG. 5

EP 3 480 615 A1

FIG. 6

FIG. 7

Europäisches
Patentamt

European
Patent Office

Office européen
des brevets

# EUROPEAN SEARCH REPORT

Application Number

EP 18 19 8496

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HENRIC RYDEN ET AL: "T1-weighted two-point fat/water separated PROPELLER acquired with dual bandwidths", PROCEEDINGS OF THE INTERNATIONAL SOCIETY FOR MAGNETIC RESONANCE IN MEDICINE, 25TH ANNUAL MEETING AND EXHIBITION, HONOLULU, HI, USA, 22-27 APRIL 2017, vol. 25, 1017, 7 April 2017 (2017-04-07), XP040688585, * page 1; figures 1, 2 * | 1-6,8-14 | INV. G01R33/48 |
| X | US 2016/334483 A1 (KOBAYASHI NAOHARU [US] ET AL) 17 November 2016 (2016-11-17) * paragraphs [0019], [0020], [0026]; figure 1 * | 1,6-9, 14,15 | |
| X | HAITAO ZHU ET AL: "Correction of chemical shift misregistration by images from two different bandwidths", MAGNETIC RESONANCE IMAGING, vol. 30, no. 4, 6 February 2012 (2012-02-06), pages 583-588, XP055564349, TARRYTOWN, NY, US ISSN: 0730-725X, DOI: 10.1016/j.mri.2011.12.009 * page 583 - page 586; figure 3; tables 1, 2 * | 1,2,6,9, 10,14 | |

| | | | TECHNICAL FIELDS SEARCHED (IPC) |
|---|---|---|---|
| | | | G01R |

The present search report has been drawn up for all claims

| Place of search | Date of completion of the search | Examiner |
|---|---|---|
| Munich | 8 March 2019 | Raguin, Guy |

EPO FORM 1503 03.82 (P04C01)

## ANNEX TO THE EUROPEAN SEARCH REPORT
## ON EUROPEAN PATENT APPLICATION NO.

EP 18 19 8496

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2019

| Patent document cited in search report | Publication date | Patent family member(s) | Publication date |
|---|---|---|---|
| US 2016334483 A1 | 17-11-2016 | DE 102016005916 A1<br>US 2016334483 A1 | 17-11-2016<br>17-11-2016 |

EPO FORM P0459

For more details about this annex : see Official Journal of the European Patent Office, No. 12/82